# DEMANDE DE BREVET EUROPEEN

(11) **EP 1 925 624 A1**
(43) Date de publication de la demande: **28.05.2008**
(21) Numéro de dépôt: 08100379.0
(22) Date de dépôt: 23.06.2005
(51) Int. Cl.: C07H 19/073, A61K 31/706

(54) **Utilisation de l'acide squalénique pour la formulation d'un principe actif à l'état de nanoparticules**

(30) Priorité: 30.06.2004 FR 0451365
(62) Demande divisionnaire de: 05857322.1
(71) Demandeur: Centre National de la Recherche Scientifique, 75794 Paris Cedex 16 (FR); UNIVERSITE PARIS-SUD (PARIS 11), 91405 Orsay (FR); Rocco, Flavio, 10042 Nichelino TO (IT)
(72) Inventeur: Rosilio, Véronique, 91140, VILLEBON-SUR-YVETTE (FR); Renoir, Jack-Michel, 94240, L'HAY LES ROSES (FR); Couvreur, Patrick, 91140, VILLEBON-SUR-YVETTE (FR); Rocco, Flavio, 10042, NICHELINO TO (IT); Cattel, Luigi, 10133, TORINO (IT); Stella, Barbara, 10134, TORINO (IT)
(74) Mandataire: Le Coupanec, Pascale A.M.P.

(57) **Abrégé**

La présente invention concerne l'utilisation de l'acide squalénique ou d'un dérivé de celui-ci pour formuler au moins un principe actif de nature polaire et de poids moléculaire supérieur à 100 Da à l'état de nanoparticules.

Elle vise également les nanoparticules ainsi obtenues et des compositions comprenant au moins lesdites nanoparticules.

## Description

La présente invention vise à proposer de nouveaux dérivés de la 2',2'-difluoro-2'-désoxycytidine (gemcitabine) particulièrement intéressants pour leur aptitude à s'organiser à l'état de nanoparticules.

La gemcitabine est un agent anti-cancéreux, analogue de la désoxycytidine, actif contre les tumeurs solides de type cancer du colon, du poumon, du pancréas, du sein, de la vessie, des ovaires, etc. (Hertel L.W. et al., Cancer Res., 50 ; 1990, 4417-4422 et Plunkett W. et al., Anticancer Drugs, 6 (Suppl. 6) ; 1995, 7-13). Sa structure chimique est similaire à celle de la cytarabine (Ara-C), à l'exception de la présence de deux atomes de fluor géminaux localisés en position 2' du sucre désoxyribose. Cette différence structurale a pour effet avantageux d'accroître la lipophilie et la perméation membranaire de la molécule de gemcitabine comparativement à celle de la cytarabine, et donc de lui conférer une toxicité plus élevée (Heinemann V. et al., Cancer Res., 48 ; 1988, 4024-4031).

Le mécanisme d'action de la gemcitabine peut être explicité-comme suit.

La gemcitabine est activée au niveau intracellulaire par phosphorylation en position 5' par action de la désoxycytidine-kinase, et ainsi transformée en son dérivé triphosphate. Celui-ci est alors incorporé dans la chaîne de l'ADN en réplication avec comme conséquence l'arrêt de l'élongation de la chaîne et la mort de la cellule (Plunkett W. et al., Semin. Oncol., 22 (4 Suppl. 11) ; 1995, 3-10).

Toutefois, la gemcitabine est parallèlement métabolisée, par action de la désoxycytidine déaminase, localisée principalement dans le sang, le foie et le rein, en son dérivé uracile qui s'avère totalement inactif (Heinemann V. et al., Cancer Res., 52 ; 1992, 533-539). En conséquence, lorsque la gemcitabine est administrée par voie intraveineuse, elle possède une activité anticancéreuse non optimale compte tenu de sa demi-vie plasmatique significativement réduite (Storniolo A.M. et al., Semin. Oncol., 24 (2 Suppl. 7) ; 1997, S7-2-S7-7).

Pour protéger la gemcitabine contre cette déamination, il a été proposé de coupler de manière covalente son radical amino en position 4 avec une chaîne acyclique. Cette alternative est notamment considérée dans le brevet EP 986 570 qui décrit des esters et amides de la gemcitabine dans lesquels les groupements 3'- et/ou 5'-OH et/ou le groupement N4-aminé sont dérivatisés avec des chaînes saturées ou mono-insaturées en C₁₈ à C₂₀. Cependant, l'augmentation d'activité anticancéreuse observée avec ces dérivés lipophiles comparativement à la gemcitabine est, dans ce cas, obtenue au détriment de leur solubilité en milieu aqueux. En effet, compte tenu de leur caractère très lipophile, ces dérivés s'avèrent difficilement compatibles avec une administration intraveineuse.

La présente invention a précisément pour objet de proposer de nouveaux dérivés de la gemcitabine dotés d'une activité anticancéreuse supérieure à la gemcitabine en raison d'une stabilité significative à la métabolisation et d'une demi-vie plasmatique prolongée et qui soient néanmoins compatibles avec une administration parentérale et notamment intraveineuse.

Plus précisément, la présente invention concerne selon un premier aspect, un dérivé de 2',2'-difluoro-2'-désoxycytidine de formule générale (I) : 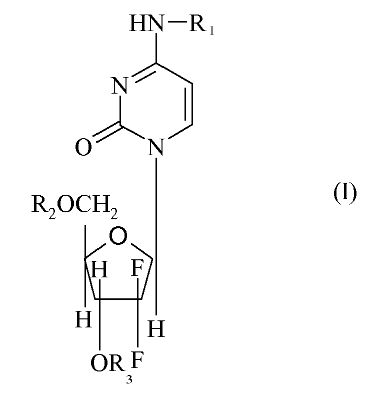 dans laquelle :
R₁, R₂ et R₃, identiques ou différents, représentent, indépendamment l'un de l'autre, un atome d'hydrogène ou un radical acyle hydrocarboné au moins en C₁₈ et de conformation telle qu'il est capable de conférer au dit composé de formule générale (I), une forme compactée notamment de type nanoparticulaire, dans un milieu solvant polaire, l'un au moins des groupements R₁, R₂ et R₃ étant différent d'un atome d'hydrogène.

La présente invention concerne selon un autre de ses aspects, des nanoparticules de dérivés de gemcitabine conformes à la présente invention.

La présente invention concerne selon un autre de ses aspects, un procédé de préparation de ces nanoparticules, caractérisé en ce qu'il comprend :
- la solubilisation d'un dérivé de la gemcitabine selon l'invention dans au moins un solvant organique à une concentration suffisante pour obtenir, lors de l'ajout du mélange résultant, sous agitation, à une phase aqueuse, la formation instantanée de nanoparticules dudit dérivé en suspension dans ladite phase aqueuse, et, le cas échéant,
- l'isolement desdites nanoparticules.

La présente invention concerne également selon un autre de ses aspects, l'utilisation de ces dérivés et nanoparticules pour la préparation d'une composition pharmaceutique dotée d'une activité anticancéreuse ou antivirale.

Elle concerne en outre une composition pharmaceutique comprenant, à titre de matière active, au moins un dérivé conforme à la présente invention, notamment sous la forme de nanoparticules.

Enfin, elle concerne également l'utilisation de l'acide squalénique ou de l'un de ses dérivés pour formuler un principe actif de nature polaire avec un poids moléculaire supérieur ou égale à 100 Da, en particulier supérieur à 150 Da, plus particulièrement supérieur à 200 Da, et notamment un nucléoside ou analogue à l'état de nanoparticules. Cette utilisation implique notamment le couplage, en particulier covalent, d'au moins une molécule d'acide squalénique ou de l'un de ses dérivés, à une molécule de principe actif considéré.

De manière inattendue, les inventeurs ont ainsi constaté que le couplage covalent de la gemcitabine avec au moins une molécule d'un dérivé hydrocarboné, au moins en C₁₈, permettait, sous réserve que ce dérivé hydrocarboné possède une conformation apte à s'organiser sous une forme compactée dans un milieu solvant polaire, d'accéder à des nanoparticules à base de gemcitabine.

Les dérivés de gemcitabine sont avantageux à plusieurs titres.

De par leur caractère très hydrophobe et insoluble dans l'eau, ils sont capables de s'organiser spontanément à l'état de particules par nanoprécipitation.

Compte tenu de la faible taille de leurs particules, ils sont administrables sous la forme d'une suspension aqueuse par voie intraveineuse et donc compatibles avec la microcirculation vasculaire.

Les dérivés hydrocarbonés en C₁₈ considérés selon l'invention sont généralement fixés par une liaison covalente au niveau d'un groupement 3'- et/ou 5'-OH et/ou du groupement 4-amino de la gemcitabine.

Conviennent tout particulièrement à l'invention, les dérivés hydrocarbonés acycliques, non linéaires et insaturés notamment à l'image des dérivés terpéniques comme par exemple le squalène et ses dérivés.

Avantageusement, ce dérivé hydrocarboné est un acide carboxylique. Auquel cas, cette liaison covalente est plus particulièrement de nature ester dans le cas des groupements 3' ou 5'-OH, et amide dans le cas du groupement 4-amino.

Bien entendu, les dérivés de gemcitabine selon la présente invention peuvent être des dérivés comprenant deux dérivatisations, voire trois dérivatisations, celles-ci pouvant être identiques ou différentes.

Selon une variante particulière de l'invention, les dérivés selon l'invention possèdent au moins un dérivé acyle hydrocarboné au niveau du groupement 4-amino. Il est alors figuré par le radical R₁.

Plus particulièrement, les dérivés de gemcitabine de l'invention répondent à la formule générale (IA) : 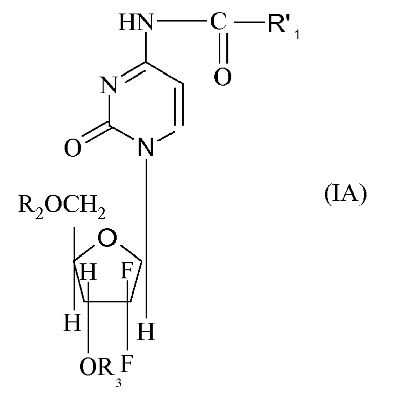 dans laquelle R₂ et R₃ sont tels que définis précédemment et R'₁ représente un radical squalénoyle ou dérivé de celui-ci.

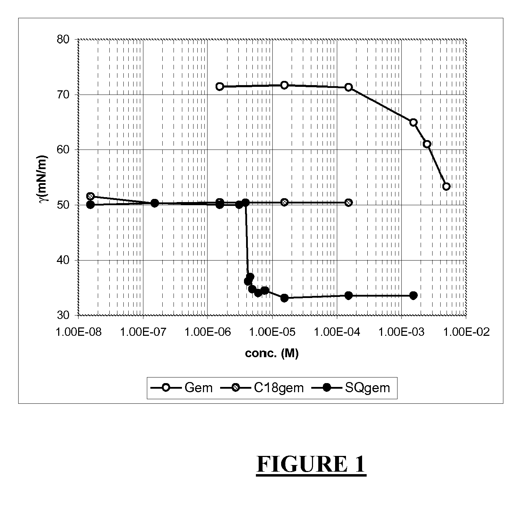

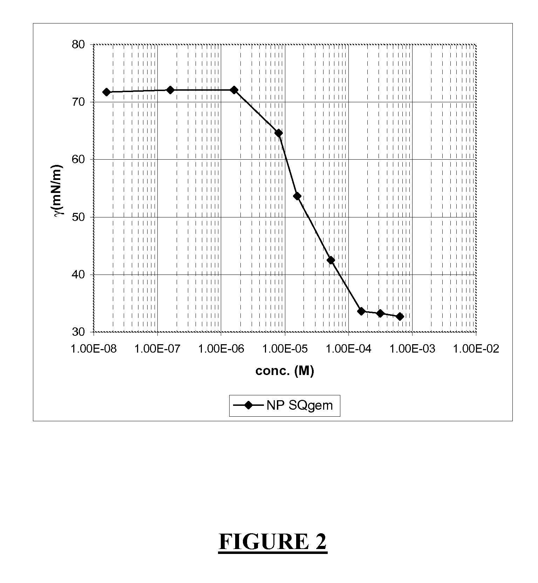

Au sens de l'invention, le terme dérivé de squalénoyle entend couvrir les dérivés de substitution du radical squalénoyle dans la mesure où la présence de ce ou ces substituant(s) n'a pas d'incidence significative sur la conformation d'origine du radical. En d'autres termes, le radical doit conserver son aptitude à se compacter ou encore à provoquer une diminution significative de la tension superficielle ou encore chute rapide de la tension superficielle, lorsqu'il est mis en présence, à partir d'une certaine concentration, avec un solvant polaire. Ce phénomène est notamment illustré en figures 1 ou 2.

Plus particulièrement, R₂ et R₃ peuvent représenter alors un atome d'hydrogène.

La présente invention concerne tout particulièrement le dérivé 4-(*N*)-squalénoylgemcitabine (SQgem).

De manière inattendue, les inventeurs ont ainsi constaté que les dérivés conformes à la présente invention et comprenant, à titre de radical hydrocarboné un radical squalénoyle, s'avéraient particulièrement sensibles à la polarité des solvants, à l'image du squalène. Ils ont ainsi constaté que la mise en présence de ces dérivés de gemcitabine avec un solvant polaire à l'image de l'eau par exemple, conduisait à la formation spontanée de particules à l'échelle du nanomètre et donc avantageusement compatibles avec une administration intraveineuse.

Les nanoparticules des dérivés de gemcitabine selon la présente invention s'avèrent accessibles à l'aide de la technologie classique de nanoprécipitation telle que décrite dans Fessi H. et al., Int. J. Pharm., 55 ; 1989, R1-R4.

Plus précisément, les nanoparticules selon l'invention sont obtenues par solubilisation d'un dérivé conforme à la présente invention dans un solvant organique à l'image de l'acétone et/ou de l'éthanol. L'ajout du mélange, ainsi obtenu, dans une phase aqueuse sous agitation conduit instantanément à la formation des nanoparticules attendues en présence ou non de tensioactif(s).

De manière avantageuse, le procédé ne requiert pas la présence obligatoire de tensioactif(s) pour obtenir des nanoparticules conformes à l'invention. Cette propriété est particulièrement appréciable dans la mesure où un grand nombre de tensioactifs ne s'avèrent pas compatibles avec une application in vivo.

Toutefois, il est entendu que l'usage de tensioactifs, généralement avantageusement dénués de toute toxicité, est envisageable dans le cadre de la présente invention. Ce type de tensioactif peut par ailleurs permettre d'accéder à des tailles encore plus réduites lors de la formation des nanoparticules.

A titre illustratif et non limitatif de ce type de tensioactifs susceptibles d'être utilisés dans la présente invention, on peut notamment citer des copolymères de polyoxyéthylène-polyoxypropylène, des dérivés phospholipidiques et des dérivés lipophiles du polyéthylène glycol. Comme dérivé lipophile du polyéthylène glycol, on peut mentionner par exemple le polyéthylène glycol cholestérol.

Comme exemple des copolymères blocs polyocyéthylène-polyoxypropylène, on peut plus particulièrement citer les copolymères triblocs polyoxyéthylène-polyoxypropylène-polyoxyéthylène, encore appelés Poloxamers^{®}, Pluronics^{®} ou Synperonics^{®}, et qui sont commercialisés notamment par la société BASF. Apparentés à ces familles de copolymères, les Poloxamines^{®}, qui sont constituées de segments hydrophobes (à base de polyoxypropylène), de segments hydrophiles (à base de polyoxyéthylène) et d'une partie centrale dérivant du motif éthylène diamine, peuvent également être employés.

La suspension colloïdale de particules peut être conservée telle quelle, voire évaporée de manière à concentrer les nanoparticules selon l'invention.

D'une manière générale, les nanoparticules ainsi obtenues possèdent une taille moyenne en poids variant de 30 à 500 nm, et en particulier de 50 à 250 nm, notamment de 70 à 200 nm, et voire de 100 à 175 nm mesurée par diffusion de la lumière à l'aide du nanosizer Coulter^{®} N4MD, Coulter Electronics, Hialeah, USA.

Cette aptitude des dérivés selon l'invention à conduire à la formation de nanoparticules, est de toute évidence le résultat d'un comportement spécifique de ces dérivés en milieu aqueux. Ainsi, comme il ressort des exemples figurant ci-après, un dérivé 4-(*N*)-squalénoylgemcitabine s'avère posséder un comportement très différent de la gemcitabine ou d'un dérivé 4-(*N*)-stéaroylgemcitabine en milieu aqueux. Seul le dérivé selon l'invention conduit à une diminution significative de la tension superficielle de l'eau.

Les inventeurs ont en outre constaté qu'il était possible de contrôler la taille de ces particules à travers la quantité de dérivé de gemcitabine mise en oeuvre pour la nanoprécipitation. En effet, l'augmentation de la concentration de la 4-(*N*)-squalenoylgemcitabine entraîne généralement une augmentation de la taille, et inversement, comme il ressort de l'exemple 2 ci-après. En outre, et comme mentionné précédemment, cette taille peut être également contrôlée en conduisant la formation des nanoparticules en présence de tensioactif(s).

Les dérivés conformes à l'invention s'avèrent par ailleurs dotés d'une activité anti-tumorale très supérieure à celle de la gemcitabine. Ainsi, les résultats présentés ci-après montrent clairement que les nanoparticules de 4-(*N*)-squalenoylgemcitabine s'avèrent cinq à sept fois plus toxiques que la molécule de gemcitabine.

Comme précisé précédemment, les composés selon l'invention s'avèrent donc avantageux à plusieurs titres, tout d'abord la fonctionnalisation de la gemcitabine en position 4-amino permet de protéger efficacement la fonction amine de l'action de la désoxycytidine déaminase, qui est naturellement responsable de la demi-vie plasmatique réduite de la gemcitabine une fois administrée par voie intraveineuse.

Toutefois, cette protection peut être en parallèle efficacement levée *in vivo* grâce à l'action des enzymes cellulaires et conduire à la libération de la gemcitabine.

Par ailleurs, la conjugaison de la gemcitabine avec un dérivé hydrocarboné conforme à l'invention, et plus particulièrement avec l'acide squalénique, confère à la molécule de la gemcitabine des caractéristiques physico-chimiques suffisantes pour lui conférer une aptitude à former des particules par nanoprécipitation, particules dont la taille s'avère compatible pour une administration parentérale et notamment par voie intraveineuse.

Les dérivés conformes à la présente invention peuvent également être administrés par toutes les voies conventionnelles. Toutefois, comme précisé précédemment ces compositions sont particulièrement intéressantes notamment lorsqu'elles sont sous la forme nanoparticulaire pour une administration parentérale.

Un autre aspect de l'invention concerne donc une composition pharmaceutique comprenant au moins, au titre de matière active, un composé conforme à la présente invention notamment sous la forme de nanoparticules. Les dérivés conformes à la présente invention peuvent y être associés avec au moins un véhicule pharmaceutiquement acceptable.

A titre d'exemples de formulations pharmaceutiques compatibles avec les compositions selon l'invention on peut notamment citer :
- les injections ou perfusions intraveineuses ;
- les solutions salines ou d'eau purifiée ;
- les compositions pour inhalation ;
- les compositions pour voie oculaire ;
- les capsules, dragées et cachets incorporant notamment à titre de véhicules de l'eau, du phosphate de calcium, des sucres, tels que lactose, dactrose ou mannitol, du talc, de l'acide stéarique, de l'amidon, du bicarbonate de sodium et/ou de la gélatine.

Lorsque les composés sont utilisés en dispersion dans une solution aqueuse, ils peuvent être associés à des excipients de type agent séquestrant ou chélatant, antioxydant, agents modifiant le pH et/ou agents tampons.

Les nanoparticules selon l'invention sont bien entendu susceptibles de porter en surface une multitude de fonctions réactives, à l'image des fonctions hydroxyles ou amines par exemple. Il est donc envisageable de fixer à ces fonctions toutes sortes de molécules, notamment par des liaisons covalentes.

A titre illustratif et non limitatif de ce type de molécules susceptibles d'être associées aux nanoparticules, on peut notamment citer les molécules de type marqueur, les composés susceptibles d'assurer une fonction de ciblage, ainsi que tous composés aptes à leur conférer des caractéristiques pharmacocinétiques particulières. En ce qui concerne ce dernier aspect, on peut ainsi envisager de fixer en surface de ces nanoparticules des dérivés lipophiles du polyéthylène glycol, comme par exemple le polyéthylène glycol cholestérol ou le polyéthylène glycol-phosphatidiléthanolamine. Un enrobage de surface à base d'un tel composé est en effet avantageux pour conférer une rémanence vasculaire accrue en raison d'une réduction significative de la capture des nanoparticules par les macrophages hépatiques.

Il est en outre possible d'envisager l'association par voie non covalente de composés conformes à la présente invention et/ou des nanoparticules correspondantes avec des molécules annexes telles que définies précédemment. Cette association peut par exemple relever de phénomènes d'adsorption dus notamment à une affinité entre les composés selon l'invention et ces autres molécules.

Ainsi, et comme illustré en exemple 4, le polyéthylène glycol sous une forme conjuguée avec du cholestérol peut être associé à une molécule conforme à la présente invention. En effet, compte tenu de l'affinité naturelle du squalène pour le cholestérol, le conjugué polyéthylène glycol cholestérol s'associe, en l'espèce, avec un conjugué squalène-gemcitabine active, et conduit ainsi à la formation de nanoparticules revêtues en surface de polyéthylène glycol. Par ailleurs, et comme mentionné précédemment, le conjugué polyéthylène glycol cholestérol agit avantageusement, lors du processus de formation des nanoparticules de squalène-gemcitabine comme un tensioactif du fait de son comportement amphiphile et stabilise donc la solution colloïdale réduisant ainsi la taille des nanoparticules formées.

Outre les composés précités, les compositions pharmaceutiques selon l'invention peuvent contenir des agents de type conservateurs, des agents mouillants, des agents solubilisants, des agents de coloration, et les parfums.

Pour des raisons évidentes, les quantités en dérivés selon l'invention susceptibles d'être mis en oeuvre notamment à des fins anti-cancéreux sont susceptibles de varier significativement selon le mode d'utilisation et la voie retenue pour leur administration.

Par exemple pour un traitement par voie systémique, destiné à un patient de type adulte, on peut envisager d'administrer un dérivé conforme pour la présente invention à une dose d'environ 0,1 à 150 mg/kg de poids corporel et par jour et plus particulièrement de 1 à 40 mg/kg par jour.

En revanche pour une administration topique on peut envisager de formuler au moins un dérivé conforme à la présente invention à raison de 0,1 à 40 % en poids, voire plus, par rapport au poids total de la formulation pharmaceutique considérée.

Il est également possible de co-administrer au moins un dérivé conforme à la présente invention avec au moins une autre matière active susceptible d'être également bénéfique à l'égard de la pathologie considérée.

A titre représentatif de ces matières actives, susceptibles d'être combinées aux dérivés conformes à la présente invention, on peut notamment citer d'autres molécules ou macromolécules anticancéreuses ou cytostatiques (par exemple sels de platine, antracyclines, poisons du fuseau mitotique, inhibiteurs de topoisomérases, de kinases ou de métalloprotéases), des agents anti-inflammatoires de type corticoïde (par exemple dexamétasone) ou non corticoïde ou encore des molécules à activité immunoadjuvantes (par exemple anticorps à activité anticancéreuse). L'association avec l'hyperthermie utilisée dans certaines chimiothérapies peut être envisagée. Les dérivés conformes à la présente invention peuvent également être combinés aux thérapies chirurgicales et/ou aux radiations pour le traitement du cancer.

Un autre aspect de l'invention concerne l'utilisation de l'acide squalénique ou de l'un de ses dérivés pour formuler un principe actif de nature polaire avec un poids moléculaire supérieur ou égale à 100 Da, en particulier supérieur à 150 Da, plus particulièrement supérieur à 200 Da, et notamment un nucléoside ou analogue à l'état de nanoparticules. En effet, les inventeurs ont montré que des nucléosides antiviraux associés de façon covalente à un dérivé squalénique pouvaient former des nanoparticules. Cet aspect est plus particulièrement illustré par les exemples 6, 7 et 8.

A titre illustratif et non limitatif des nucléosides antiviraux ou analogues structuraux susceptibles d'être formulés selon l'invention, on peut notamment citer la didanosine, la zidovudine et l'acyclovir mais aussi la zalcitabine, le gancyclovir, le valacyclovir, la stavudine, la lamivudine, l'abacavir, l'emtricitabine, l'amdoxovir, le 2'-déoxy-3'-oxa-4'-thiocytidine (dOTC) ou même le sidophovir.

Les exemples et figures présentés ci-après sont soumis à titre illustratif et non limitatif du domaine de l'invention.

### Figures

*Figure 1* : Elle représente l'évolution de la tension superficielle (γ) de solutions de gemcitabine (Gem), 4-(*N*)-stéaroylgemcitabine (C18gem) et 4-(*N*)-squalénoylgemcitabine (SQgem) en fonction de la concentration des solutions.
*Figure 2* *:* Elle représente l'évolution de la tension superficielle (γ) d'une suspension de nanoparticules de SQgem (NP SQgem) en fonction de la concentration.
*Figure 3* *:* Elle représente la viabilité cellulaire en fonction du temps après incubation de nanoparticules de SQgem 100 µM sur deux lignées cellulaires en présence et en absence d'inserts (test MTT) (n = 3).

### Exemple 1 : Préparation de la 4-(N)-squalénoylgemcitabine (SQgem)

### a) Synthèse de l'acide squalénique (SQCOOH)

A 11 ml d'eau distillée sont ajoutés 1,16 ml d'acide sulfurique; ensuite, 0,615 g (2,06 mmol) de Na₂Cr₂O₇·2H₂O sont ajoutés doucement afin d'obtenir l'acide chromique. 0,794 g (2,06 mmol) de l'aldéhyde squalénique (SQCHO) (Ceruti M*. et al., J.* Chem. Soc., Perkin Trans, 1 ; 2002, 1477-1486) sont dissous dans 16 ml d'éther diéthylique sous agitation magnétique et le ballon est ensuite refroidi à 0° C. Ensuite, à la solution de SQCHO est ajouté l'acide chromique goutte à goutte. La réaction est maintenue sous agitation magnétique à 0° C pendant deux heures. Le produit brut est ensuite purifié par lavage de la phase organique avec de l'eau et ensuite par flash-chromatographie sur gel de silice avec éther de pétrole/éther 95:5 comme éluent. Rendement: 35 % (0,286 g, 0,714 mmol).

¹H RMN (CD₃COCH₃ 99.5% 300 MHz) δ:5.11 (5H, m, CH vinyliques), 2,38 (2H, t, CH₂CH₂COOH), 2,26 (2H, t, CH₂CH₂COOH), 2,13-1,86 (16H, m, CH₂ allyliques), 1,65-1,59 (15H, m, CH₃ allyliques), 1.26 (3H, s, CH₃ allyliques).

CIMS (isobutane) *m*/*z* 401 (100).

EIMS *mlz* 400 (5), 357 (3), 331 (5), 289 (3), 208 (6), 136 (3), 81 (100).

### b) Synthèse de la 4-(N)-squalénoylgemcitabine

Dans un ballon à trois cols muni d'un fluxmètre, 0,209 g (0,522 mmol) de SQCOOH obtenus en a) dissous dans 1 ml tétrahydrofurane (THF) anhydre et ensuite 0,053 g (0,522 mmol) de triéthylamine (TEA) dissous dans 0,5 ml THF anhydre sont ajoutés sous agitation magnétique et avec un flux d'argon. Le ballon est ensuite refroidi à -15°C. Au mélange de réaction, 0,057 g (0,522 mmol) d'éthylchloroformiate dissous dans 2,15 ml THF anhydre sont ajoutés goutte à goutte. Après 20 minutes à -15° C, 0,137 g (0,522 mmol) de gemcitabine dissous dans 2,72 ml de diméthylformamide (DMF) sont ajoutés et la température est augmentée jusqu'à +5°C et finalement à température ambiante. La réaction est suivie par chromatographie sur couche mince (dichlorométhane/acétone 50:50) et elle est gardée sous agitation magnétique pendant plusieurs jours jusqu'à la formation de l'amide. Le produit brut est ensuite purifié par flash-chromatographie sur gel de silice avec un mélange dichlorométhane/acétone 95:5 comme éluent. Rendement: 55 % (0,185 g, 0,287 mmol).

¹H RMN (pyridine-d₅ 99.5% 300 MHz) δ: 12,05 (1H, s, NHCO), 8,77 (1H, d, CH-6), 7,74 (1H, d, CH-5), 6,99 (1H, t, CH-1'), 5,30-5,02 (1H, m, CH-3' et 5H, m, CH vinyliques), 4,47-4,31 (3H, m, CH-4' et CH₂-5'), 2,81 (2H, t, NHCO*CH2*), 2,53 (2H, t, NHCOCH₂*CH₂*), 2,18-2,00 (16H, m, CH₂ allyliques), 1,68-1,55 (18H, m, CH₃ allyliques).

CIMS (isobutane) *m*/*z* 646 (100).

EIMS *m*/*z* 645 (10), 577 (8), 523 (7), 509 (18), 494 (10), 454 (15), 429 (24), 372 (100).

### c) Préparation des nanoparticules constituées de 4-(N)-squalénoylgemcitabine

Les particules constituées de SQgem sont obtenues selon la technique de la nanoprécipitation décrite dans Fessi H. et al, Int. J. Pharm., 55 ; 1989, R1-R4. Un échantillon d'une solution à 10 mg/ml de SQgem dans l'éthanol est prélevé et ajouté à de l'acétone selon la concentration désirée et d'une façon telle à obtenir un total de 2 ml de phase organique. Cette solution de SQgem dans le mélange éthanol/acétone est ensuite ajoutée à 4 ml d'eau MilliQ^{®} sous agitation magnétique. Les particules se forment instantanément. Après évaporation sous vide des solvants organiques, une suspension de particules colloïdales stables de SQgem est obtenue. La suspension doit être conservée à +4° C.

### Exemple 2 : Caractérisation physico-chimique des particules

### a) Détermination de la taille des nanoparticules

Le contrôle de la taille des particules colloïdales obtenues en exemple 1 est effectué par diffusion quasi élastique de la lumière avec un nanosizer (Coulter^{®} N4MD, Coulter Electronics, Hialeah, USA).

Les suspensions colloïdales sont diluées dans de l'eau MilliQ^{®} afin que le nombre de particules par ml soit adapté à l'appareillage de mesure.

La taille des nanoparticules est comprise entre 100 et 200 nm. Elle est contrôlée par différentes concentrations en SQgem mises en oeuvre dans le procédé de nanoprécipitation décrit en exemple 1. Les résultats obtenus sont présents en tableau I ci-après.

**Tableau 1**

| Concentration de SQgem dans la suspension finale (mg/ml) | Diamètre hydrodynamique moyen (nm) | Ecart-type | Index de polydispersité |
|---|---|---|---|
| 1 | 126,2 | 43,5 | 0,26 |
| 2 | 150,4 | 37,7 | 0,09 |
| 4 | 171,3 | 40,8 | 0,08 |

### b) Mesure de la tension superficielle de solutions de 4-(N)-squalénoylgemcitabine et étude de leur stabilité

La tension superficielle d'une solution aqueuse de SQgem à différentes concentrations est mesurée, à surface constante, à l'aide d'un tensiomètre à lame de Wilhelmy et comparée à celle d'une solution de gemcitabine (Gem) et de *4-(N)-*stéaroylgemcitabine (C18gem) (Myhren F*. et al.,* Gemcitabine derivatives, US patent n° 2002/0042391).

Pour préparer les différentes dilutions de SQgem et de C18gem il est nécessaire de partir d'une solution éthanolique; le pourcentage d'éthanol dans la solution finale est de 10 % (la présence d'éthanol abaisse la tension superficielle de l'eau de 72 mN/m à environ 50 mN/m). Les résultats sont présentés en figure 1.

On note que seule la SQgem diminue la tension superficielle à une concentration de 4×10⁻⁶ M, ce qui correspond exactement à la formation des nanoparticules.

La stabilité des particules de SQgem à la dilution a également été évaluée par la mesure de la tension superficielle à aire constante de différentes dilutions d'une suspension de particules de SQgem. Elle est illustrée par la figure 2.

### Exemple 3 : Détermination de l'activité antitumorale des particules de 4-(N)-squalénoylgemcitabine

L'activité cytotoxique de la SQgem est évaluée sur deux lignées cellulaires tumorales humaines (KB3-1, cancer du naso-pharynx et MCF-7, cancer du sein) par exposition à la SQgem pendant 72 heures et comparée à l'activité de la gemcitabine.

Les lignées sont maintenues en milieu DMEM avec 10 % de sérum de veau fétal, glutamine 2 mM, antibiotiques 50 mg/l, à 37° C, 5 % CO₂, 95 % d'humidité. Les cellules sont ensemencées dans une plaque à 96 puits à 1x10⁴/puit; après 24 heures, différentes dilutions de gemcitabine et de particules de SQgem sont ajoutées et incubées pendant 72 heures. La viabilité cellulaire est ensuite déterminée par le test MTT. Les résultats sont exprimés en CI₅₀, qui est la concentration de molécule à laquelle 50 % des cellules sont vivantes.

Les résultats obtenus sont présentés en tableau II ci-après. Ils montrent clairement que les nanoparticules de SQgem sont 5 à 7 fois plus cytotoxiques que la molécule de gemcitabine.

**Tableau II**

| | CI₅₀ (µM) après 72 h | |
|---|---|---|
| | KB3-1 | MCF-7 |
| Gemcitabine | 50,8 ± 49,8 | 29,0 ± 13,9 |
| SQgem | 8,8 ± 4,1 | 4,8 ± 3,9 |

L'activité cytotoxique de la SQgem à temps différents a également été évaluée par incubation de nanoparticules de SQgem 100 µM sur deux lignées cellulaires en présence et en absence d'inserts (0,02 µm) (test MTT) (n = 3).

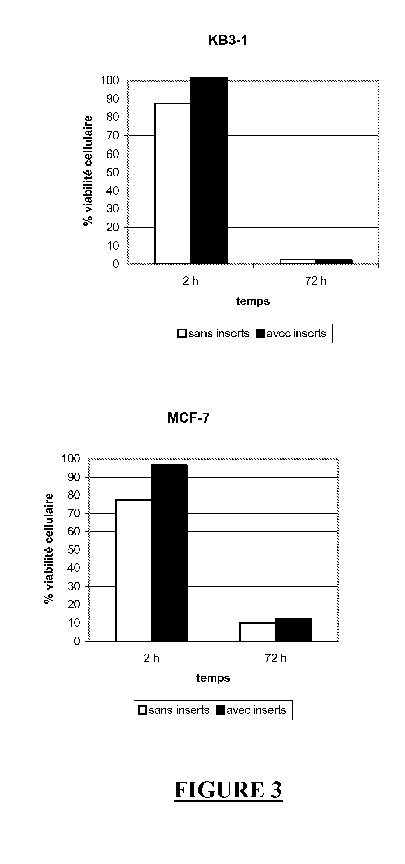

Les résultats obtenus sont présentés en figure 3. On note que l'activité anticancéreuse de la SQgem n'est pas réduite par rapport aux essais de cytotoxicité en l'absence d'insert.

### Exemple 4: Préparation de nanoparticules de 4-(N)-squalénoylgemcitabine peggylées

2 mg de 4-(N)-squalénoylgemcitabine et 1,4 mg de cholestérol couplé au polyéthylène glycol (Chol-PEG CHOLESTEROL PEGYLE, SUNBRIGHT CS-020) sont dissous dans 1 mL d'acétone. Cette phase organique est ajoutée à 2 mL d'eau MilliQ^{®} sous agitation magnétique. Après évaporation sous vide de l'acétone, une suspension de nanoparticules stables est obtenue. La taille des nanoparticules, évaluée selon le protocole décrit dans l'exemple 2, est d'environ 75 nm et le potentiel zéta de -32,7 mV.

### Exemple 5 : Préparation des nanoparticules constituées de 4-(N)-squalénoylcytarabine (SQara-C)

La 4-(*N*)-squalénoylcytarabine est synthétisée à partir de l'acide squalénique par réaction avec la cytarabine, selon la procédure décrite dans l'exemple 1 pour la 4*-*(*N*)-squalénoylgemcitabine. Les particules constituées de SQara-C sont obtenues selon la technique de la nanoprécipitation, comme décrit pour les particules de SQgem, et leur diamètre hydrodynamique moyen est de 110,4 ± 34,1 nm pour une concentration en SQara-C dans la suspension finale de 1 mg/ml (index de polydispersité : 0,168)

### Exemple 6 : Synthèse des nanoparticules de la 5'-squalenoyl-didanosine; (2S, 5R)-((4,8,13,17,21-Pentamethyl-docosa-4,8,12,16,20-pentaenoate de 5-(6-oxo-1,6-dihydro-purin-9-yl)-tétrahydro-furan-2-yl-méthyle

A une solution de 31 mg d'acide (4,8,13,17,21-pentaméthyl-docosa-4,8,12,16,20-pentaenoïque (SqCO2H, 0,15 mmol) dans le diméthylformamide anhydre (1,2 mL) sont ajoutés 28 mg de N-hydroxybenzotriazol (0,18 mmol), 36 mg de didanosine (ddI, 0,15 mmol), 70 mg de O-(7-azabenzotriazol-1-yl)-N,N,N',N'-tetramethyluronium hexafluoroborate (0,18 mmol) et finalement 62 mg de diisopropyléthylamine (0,5 mmol). Le mélange est agité 84 h à 20 °C sous atmosphère d'azote, puis concentré sous pression réduite (0,05 Torr). Le résidu est repris dans 5 mL d'une solution aqueuse saturée en bicarbonate de sodium, et extrait à l'acétate d'éthyle (3 X 10 mL). La phase organique est lavée par une solution aqueuse de NaCl, séchée sur MgSO4, et concentrée sous vide. Le résidu est chromatographié sur gel de silice (CH2Cl2/MeOH: 92/8) pour fournir 37 mg de 5'-squalenoyldidanosine (Rdt 58%) sous la forme d'un solide amorphe incolore.

IR (cm-1) 3550-2700, 2921, 2856, 1734, 1691, 1590, 1548, 1449, 1380, 1261.

¹H RMN (200 MHz, CDCl3) δ : 13.0 (s large, 1H), 8.18 (s, 1H), 8.08 (s, 1H), 6.38 (t, J = 4,2 Hz, 1H), 5,17-5,00 (m, 5H), 4,40-4,20 (m, 3H), 2,60-1,90 (m, 24 H), 1,67 (s, 3H), 1.60 (s large, 15 H).

¹³C RMN (50MHz, CDCl3) δ : 173.27 (CO2), 159.20 (CO), 148.34 (C), 144.3 (CH) 138.60 (CH), 135.23 (C), 135.03 (C), 135.00 (C), 133.09 (C), 131.31 (C), 125.56 (CH), 125.38 (C), 125.54 (CH), 124.53 (CH), 124.40 (2 CH), 85.94 (CH), 79.60 (CH), 65.07 (CH2), 39.86 (CH2), 39.83 (CH2), 39.68 (CH2), 34.67 (CH2), 33.12 (CH2), 33.01 (CH2), 28.39 (CH2), 28.38 (CH2), 29.9 (CH2), 26.83 (CH2), 26.79 (CH2), 26.28 (CH2), 25.77 (CH3), 17.77 (CH3), 16.51 (2 CH3), 16.10 (CH3), 16.00 (CH3).

Le même composé peut être obtenu avec un rendement de 10% en utilisant l'EDCI comme agent de couplage alors que la condensation entre le chlorure de l'acide squaloyle et le ddI le donne avec un rendement de 15%.

La taille des particules est évaluée selon le protocole décrit dans l'exemple 2. Le diamètre hydrodynamique moyen est de 152 nm, mesuré avec un écart type de 34,4nm et un indice de polydispersité de 0,1.

### Exemple 7 : Synthèse des nanoparticules de la 5'-squalenoyl-zidovudine; (2S,3S,5R)-4,8,13,17,21-Pentamethyl-docosa-4,8,12,16,20-pentaenoate de 3-azido-5-(5-méthyl-2,4-dioxo-3,4-dihydro-2H-pyrimidin-1-yl)-tétrahydro-furan-2-yl-méthyle

A une solution de 50 mg d'acide (4,8,13,17,21-pentaméthyl-docosa-4,8,12,16,20-pentaenoïque (SqCO2H, 0,15 mmol) dans le diméthylformamide anhydre (2 mL) sont ajoutés 45 mg de N-hydroxybenzotriazol (0,29 mmol), 79 mg de zidovudine (AZT, 0,24 mmol), 113 mg de O-(7-azabenzotriazol-1-yl)-N,N,N',N'-tetramethyluronium hexafluoroborate (0,29 mmol) et finalement 102 mg de diisopropyléthylamine (0,5 mmol). Le mélange est agité 90 h à 20 °C sous atmosphère d'azote, puis concentré sous pression réduite (0,05 Torr). Le résidu est repris dans 10 mL d'une solution aqueuse saturée en bicarbonate de sodium, et extrait à l'acétate d'éthyle (3 X 15 mL). La phase organique est lavée par une solution aqueuse de NaCl, séchée sur MgSO4, et concentrée sous vide. Le résidu est chromatographié sur gel de silice (CH2Cl2/MeOH: 97/3) pour fournir 52 mg de 5'-squalenoyl-zidovudine (Rdt 43%) sous la forme d'un solide amorphe incolore. IR (cm-1) 3158, 2920, 2854, 2105, 1741, 1690, 1449, 1381, 1270.

¹H RMN (200 MHz, CDCl3) δ : 8.2 (s large, 1H), 7.22 (s, 1H), 6.12 (t, J = 6.4 Hz, 1H), 5,17-5,00 (m, 5H), 4,40 (dd, J = 12.2 Hz, 4.6 Hz, 1H), 4.30 (dd, 12.2 Hz, 3.8 Hz, 1H), 4.10-4.05 (m, 1H), 2,55-2.20 (m, 5 H), 2.10-1.90 (m, 18 H), 1.94 (s, 3H), 1,69 (s, 3H), 1.60 (s large, 15 H).

¹³C RMN (50 MHz, CDCl3) δ : 172.87 (CO2), 163.57 (CO), 150.12 (CO), 135.31 (C), 135.27 (CH), 135.04 (C), 134.91 (C), 132.86 (C), 131.35 (C), 125.79 (CH), 124.67 (CH), 124.56 (CH), 124.40 (CH), 124.37 (CH), 111.43 (C), 85.64 (CH), 82.00 (CH), 63.36 (CH2), 60.81 (CH), 39.88 (CH2), 39.85 (CH2), 39.68 (CH2), 37.75 (CH2), 34.62 (CH2), 33.18 (CH2), 29.81 (CH2), 28.41 (CH2), 28.39 (CH2), 26.91 (CH2), 26.82 (CH2), 26.81 (CH2), 25.80 (CH3), 17.79 (CH3), 16.17 (2 CH3), 16.16 (CH3), 16.12 (CH3), 16.05 (CH3), 12.73 (CH3).

La taille des particules est évaluée selon le protocole décrit dans l'exemple 2. Le diamètre hydrodynamique moyen est de 150-170 nm.

### Exemple 8: Préparation des nanoparticules constituées de 4-(N)-squalénoylacyclovir (SOACV)

Le 4-(*N*)-squalénoylacyclovir est synthétique à partir de l'acide squalénique par réaction avec l'acyclovir. Selon la procédure utilisée, il est possible d'obtenir entre la chaîne squalénique et l'acyclovir soit une liaison ester, soit une amidique. Les particules contituées de SQACV sont obtenues selon la technique de la nanoprécipitation, comme décrit pour les particules de SQgem, et leur diamètre hydrodynamique moyen est de 217,5 + 37,9 nm pour une concentration en SQACV dans la suspension finale de 1 mg/ml (index de polydispersité : 0,038).

## Revendications

1. Utilisation de l'acide squalénique ou d'un dérivé de celui-ci pour formuler au moins un principe actif de nature polaire et de poids moléculaire supérieur à 100 Da à l'état de nanoparticules.

2. Utilisation selon la revendication 1, **caractérisée en ce que** le poids moléculaire est supérieur à 150 Da.

3. Utilisation selon l'une des revendications 1 ou 2, **caractérisée en ce que** le poids moléculaire est supérieur à 200 Da.

4. Utilisation selon l'une quelconque des revendications précédentes, dans laquelle au moins une molécule dudit acide squalénique ou d'un dérivé de celui-ci est couplée de manière covalente à une molécule dudit principe actif.

5. Utilisation selon l'une quelconque des revendications précédentes, **caractérisée en ce que** ladite molécule de principe actif liée de manière covalente à au moins une molécule d'acide squalénique ou d'un dérivé de celui-ci est formulée à l'état de nanoparticules.

6. Utilisation selon la revendication précédente, dans laquelle lesdites nanoparticules possèdent une taille moyenne variant de 30 à 500 nm, en particulier de 50 à 250 nm, notamment de 100 à 175 nm.

7. Utilisation selon l'une quelconque des revendications précédentes, **caractérisée en ce que** ledit principe actif est au moins un nucléoside ou l'un de ses analogues.

8. Utilisation selon l'une quelconque des revendications précédentes, **caractérisée en ce que** ledit principe actif est un dérivé de la 2',2'-difluoro-2'-désoxycytidine.

9. Utilisation selon l'une quelconque des revendications 1 à 7, **caractérisée en ce que** ledit principe actif est choisi parmi la cytarabine, la didanosine, la zidovudine, l'acyclovir, la zalcitabine, le gancyclovir, le valacyclovir, la stavudine, la lamivudine, l'abacavir, l'emtricitabine, l'amdoxovir, le 2'-déoxy-3'-oxa-4'-thiocytidine (dOTC) et le sidophovir.

10. Utilisation selon la revendication 9, **caractérisée en ce que** ledit principe actif est la cytarabine.

11. Utilisation selon la revendication 9, **caractérisée en ce que** ledit principe actif est la didanosine.

12. Utilisation selon la revendication 9, **caractérisée en ce que** ledit principe actif est la zidovudine.

13. Utilisation selon la revendication 9, **caractérisée en ce que** ledit principe actif est l'acyclovir.

14. Nanoparticules formées d'au moins une molécule d'acide squalénique ou d'un dérivé de celui-ci couplé de manière covalente à une molécule d'un principe actif de nature polaire et de poids moléculaire supérieur à 100 Da.

15. Nanoparticules selon la revendication 14 dans laquelle ledit principe actif est un nucléoside.

16. Nanoparticules selon la revendication 14 ou 15 étant telles que définies en revendications 6 à 13.

17. Nanoparticules de 4-(N)-squalénoyl-cytarabine.

18. Nanoparticules de 5'-squalénoyl-didanosine.

19. Nanoparticules de 5'-squalénoyl-zidovudine.

20. Nanoparticules de 4-(N)-squalénoyl-acyclovir.

21. Composition pharmaceutique comprenant à titre de matière active au moins des nanoparticules selon l'une quelconque des revendications 14 à 20.
